(19) Europäisches Patentamt
European Patent Office
Office européen des brevets

(11) EP 0 558 928 B1

(12) **FASCICULE DE BREVET EUROPEEN**

(45) Date de publication et mention de la délivrance du brevet: **15.04.1998 Bulletin 1998/16**

(51) Int Cl.[6]: **C07D 307/92**, C07C 33/14, C11B 9/00, A61K 7/46

(21) Numéro de dépôt: **93101476.5**

(22) Date de dépôt: **30.01.1993**

(54) **Ethers furaniques et leur utilisation à titre d'ingrédients parfumants**

Furanether und ihre Anwendung als Riechstoffe

Furanethers and their use as perfume

(84) Etats contractants désignés:
**CH DE ES FR GB IT LI NL**

(30) Priorité: **06.03.1992 CH 721/92**

(43) Date de publication de la demande:
**08.09.1993 Bulletin 1993/36**

(73) Titulaire: **FIRMENICH SA**
**1211 Genève 8 (CH)**

(72) Inventeurs:
- **Snowden, Roger Leslie**
  **F-74580 Viry (FR)**
- **Escher, Sina Dorothea**
  **CH-1232 Confignon (CH)**

(74) Mandataire: **Salvaterra-Garcia, Maria de Lurdes**
**Firmenich SA**
**Département des Brevets**
**Case Postale 239**
**1211 Genève 8 (CH)**

(56) Documents cités:
**EP-A- 0 170 955**
**EP-A- 0 212 254**
**EP-A- 0 403 945**
**CH-A- 299 369**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 558 928 B1

## Description

La présente invention a trait à des éthers furaniques nouveaux qui sont utiles à titre d'ingrédients parfumants. Plus particulièrement, elle concerne des composés de formule

(I)

possédant une double liaison dans l'une ou l'autre des positions indiquées par les lignes pointillées et dans laquelle le symbole R représente un atome d'hydrogène ou un radical méthyle.

Les composés (I) sont des homologues insaturés d'un composé fort apprécié en parfumerie, à savoir l'Ambrox ® (tétraméthyl perhydronaphtofuran; origine: Firmenich SA, Genève, Suisse), un ingrédient parfumant typique de l'ambregris décrit, par exemple, dans EP-A-0 170 955, ayant trait à un procédé de synthèse de ce composé par cyclisation de 1β-(2-hydroxyéthyl)-perhydro-2,5,5,8aβ-tétraméthyl-2β-trans-naphtalénol dans des conditions de réaction spécifiques. Malgré leur structure très proche de celle de l'Ambrox ®, nous avons maintenant découvert que les composés (I) possédaient des propriétés olfactives distinctes de celles de l'Ambrox® et qui pouvaient s'avérer d'un emploi plus aisé pour certains types d'applications en parfumerie.

Les composés de formule (I) possèdent plusieurs centres chiraux et peuvent, de ce fait, assumer différentes formes stéréoisomères, racémiques ou optiquement actives. Selon l'invention, on citera, à titre préférentiel, le 2,3a,4,5,5aβ, 6,7,8,9,9a-décahydro-3aα,6,6,9aα-tétraméthylnaphto[2,1-b]furane de formule

(Ia)

ainsi que les mélanges contenant une quantité prépondérante de ce composé accompagné par des quantités mineures de l'un ou plusieurs de ses isomères. Il s'est avéré que ce composé possède une note odorante de type boisé-ambré, puissante et plus boisée que celle de l'Ambrox®. Il s'agit d'une note inattendue dans la mesure où, contrairement à ce que l'on observe avec l'Ambrox®, c'est surtout la note de tête du composé (Ia) qui possède un caractère ambré bien marqué, de sorte que ce composé se révèle particulièrement utile dans la préparation de compositions parfumantes dont on désire exalter l'impact de la note ambrée lors de leur application, tout en gardant un caractère boisé de fond.

Comme il apparaîtra plus loin, la synthèse du composé de formule (Ia) peut mener à des mélanges plus ou moins riches en ce composé, contenant des quantités mineures de l'un ou plusieurs de ses isomères. Ces mélanges se sont révélés tout aussi utiles pour les applications en parfumerie selon l'invention, car ils possédaient également les caractères olfactifs désirés. Par conséquent, lorsqu'on fera référence au composé (Ia) dans les exemples d'application, on se référera implicitement également auxdits mélanges.

On citera également, à titre préférentiel, le (-)-(3aR)-1,2,3a,4,6,7,8,9,9a,9bβ-décahydro-3aα,6,6,9aα-tétraméthylnaphto[2,1-b]furane de formule

(Ib)

Ce composé exhale une odeur boisée-ambrée très puissante, plus boisée que celle de l'Ambrox® et moins ambrée-animale, une note qui s'avère aussi être plus tenace que celle de l'Ambrox®, particulièrement sur le linge.

Le 2,3a,4,5,5aβ,6,7,8,9,9a-décahydro-3aα,6,6,7α,9aα-pentaméthylnaphto[2,1-b]furane de formule

(Ic)

est aussi un composé préféré selon l'invention. Il possède une note odorante boisée-ambrée de caractéristiques similaires mais plus puissante que celle de son homologue (Ia) non-méthylé, aussi moins ambrée, et surtout moins animale et marine, que celle de l'Ambrox®.

De par leurs qualités olfactives les composés selon l'invention se prêtent à des applications de type varié, aussi bien en parfumerie fine qu'en parfumerie fonctionnelle. C'est ainsi qu'ils peuvent être employés de façon avantageuse pour la préparation de compositions et bases parfumantes, parfums et eaux de toilette, mais aussi dans le parfumage d'articles divers tels les savons, les gels de bain ou douche, les shampoings et les crèmes ou lotions après-shampoing, les désodorisants corporels ou d'air ambiant et les préparations cosmétiques. Ils se prêtent également au parfumage de détergents ou adoucissants textiles, ou encore de produits d'entretien.

Dans ces applications, les composés (I) peuvent être utilisés seuls ou, comme il est plus courant en parfumerie, mélangés à d'autres ingrédients parfumants, solvants ou adjuvants usuels. Une énumération plus détaillée de la nature de ces co-ingrédients est ici superflue, l'homme du métier étant à même de les choisir en fonction de l'effet olfactif qu'il désire obtenir. Pour ce faire il peut notamment avoir recours à des oeuvres de référence telles que le livre de S. Arctander, Perfume and Flavor Chemicals, Montclair, N.J., USA (1976). L'effet olfactif désiré conditionne également le choix des proportions dans lesquelles les composés de l'invention peuvent être utilisés pour les applications susmentionnées, proportions qui dépendent également de la nature des autres ingrédients présents dans une composition donnée.

Bien que ces proportions puissent ainsi varier dans une gamme de valeurs très étendue, on peut citer, à titre d'exemple, des concentrations de l'ordre de 0,5 à 5%, voire même 10% ou plus en poids, par rapport au poids de la composition parfumante dans laquelle ils sont incorporés. Des concentrations bien inférieures à ces valeurs seront normalement utilisées lorsque les composés (I) seront utilisés pour le parfumage des articles divers mentionnés plus haut.

Les composés de l'invention de formule

(Id)

dans laquelle le symbole R représente un atome d'hydrogène ou un radical méthyle, sont préparés selon un procédé original caractérisé en ce qu'on cyclise à l'aide d'un agent acide un allénol de formule

(II)

dans laquelle R a le sens indiqué ci-dessus.

Les allénols de formule (II) sont des composés nouveaux qui font également l'objet de l'invention et qui peuvent être préparés à partir de la β-dihydroionone et de la β-dihydroirone comme il est décrit en détail dans les exemples de préparation présentés ci-après.

L'invention sera aussi décrite en détail à l'aide d'exemples d'application en parfumerie.

Exemple 1

Préparation de 2,3a,4,5,5aβ,6,7,8,9,9a-décahydro-3aα,6,6,9aα-tétraméthylnaphto[2,1-b]furane

a) Un mélange de 4-(2,6,6-triméthyl-1-cyclohexén-1-yl)-2-butanone (107 g, 0,55 mole) et d'alcool propargylique (34 g, 0,61 mole) a été ajouté goutte à goutte pendant 1,5 h à une bouillie de poudre de KOH (230 g, 4,1 mole; Fluka) dans le THF (tétrahydrofurane, 800 ml), agitée mécaniquement et maintenue à une température de 20°C. Après 3 h, le mélange brun a été versé sur une solution froide de $NH_4Cl$ (250 g, 4,7 mole) dans $H_2O$ (800 ml) et les phases ont été séparées. L'extraction de la phase aqueuse avec de l'éther et concentration des phases organiques combinées a fourni un produit solide rouge-brun qui a été recristallisé dans l'éther de pétrole (80/100) pour fournir le 4-méthyl-6-(2,6,6-triméthyl-1-cyclohexén-1-yl)-2-hexyne-1,4-diol sous forme d'une poudre jaune (110 g).

P. f. 83-84°C; rend.: 80%
$R_f$ (cyclohexane/acétate d'éthyle 1:1): 0,32
IR($CHCl_3$): 3550, 3350 (large), 2900, 2840, 1440, 1360, 1344, 1082, 1040, 980 $cm^{-1}$
RMN($^1H$, 360MHz, $D_2O$): 1,00(s, 6H) 1,41(s, 3H) 1,50(2H); 1,57(2H); 1,61(s, 3H); 1,73(2H); 1,90(t large, J=7Hz, 2H) ; 2,19(2H); 4,30(s, 2H) δppm.
RMN($^{13}C$) : 136,3(s); 127,5(s); 89,5(s); 81,7(s); 68,3(s); 50,7(t); 43,5(t); 39,9(t) ; 35,2(s) ; 32,8(t) ; 29,3(q); 28,7(q); 23,6(t) ; 19,8(q) ; 19,6(t) δppm.
SM: 250(0, $M^+$), 232(9), 217(19), 161(37), 145(40), 133(40), 121(100), 105(79), 95(87), 81(80).

b) A un mélange du diol préparé sous a) (25 g, 0,1 mole) et d'anhydride acétique (12 g, 0,12 mole), on a ajouté goutte à goutte de la triéthylamine (12 g, 0,12 mole) à température ambiante (réaction exothermique). Après encore 15 min, le mélange refroidi a été versé sur une solution aqueuse de HCl à 10% refroidie et extrait à l'éther (3 x 100 ml). Après le traitement usuel et distillation sous vide, on a obtenu 28,3 g (rend. 97%) d'acétate de 4-hydroxy-4-méthyl-6-(2,6,6-triméthyl-1-cyclohexén-1-yl)-2-hexynyl sous forme d'un sirop incolore.

P. éb. 144-150° (temp. du bain) /7 Pa
$R_f$ (cyclohexane/acétate d'éthyle 7:3) : 0,41
IR($CHCl_3$): 3540, 3380 (large), 2900, 2850, 1722, 1440, 1368, 1350, 1220, 1100, 958 $cm^{-1}$
RMN($^1H$, 360MHz, $D_2O$): 1,00(s, 6H) 1,41(2H); 1,49(s, 3H) ; 1,56(2H); 1,60(s, 3H) 1,73(2H); 1,90(t large, J=7Hz, 2H); 2,09(s, 3H); 2,19(2H); 4,71(s, 2H) δppm.
SM 292(0, $M^+$), 274(8), 217(31), 199(27), 161(36), 143(44), 121(100), 105(71), 95(94), 81(76).

c) Une solution de 3,4-dihydro-2H-pyrane (15 g, 0,17 mole) dans l'éther diéthylique (40 ml) a été ajoutée goutte à goutte pendant 20 min à une solution agitée de l'acétate préparé sous b) (15 g, 0,051 mole) dans l'éther (70 ml) contenant de l'acide p-toluènesulfonique (0,75 g, 4 mmole) à 25°C. Après 30 min, le mélange a été lavé successivement avec une solution aqueuse saturée de $NaHCO_3$ et avec $H_2O$. Un traitement usuel et une distillation sous vide ont fourni l'acétate de 4-(tétrahydro-2(2H)-pyranyloxy)-4-méthyl-6-(2,6,6-triméthyl-1-cyclohexén-1-yl)-2-hexynyle sous forme d'une huile incolore (19 g ; pureté CG: 91%; mélange de diastéréomères 1,2:1)

P. éb. 147-157°C/7 Pa ; rend. : 89%
$R_f$ (cyclohexane/acétate d'éthyle 7:3) : 0,52
IR : 2940, 2870, 1720, 1430, 1360, 1350, 1220, 1108, 1020, 960, 860 $cm^{-1}$
RMN($^1H$, 360MHz, $D_2O$) : 1,00(s, 6H); ; 1,41(2H) ; 1,47 et 1,52(2s, 3H); 1,61(s, 3H) ; 1,45-1,90(10H) ; 1,89(t large, J=7Hz, 2H) ; 2,09 et 2,10(2s, 3H) ; 2,18(2H); 3,51(1H) ; 3,93 et 3,98(2m, 1H) ; 4,72 et 4,73(2s, 2H); 5,00 et 5,08(2m, 1H) δppm.
SM isomère majeur : 376(0, $M^+$), 199(9), 137(31), 121(16), 105(11), 95(31), 85(100), 81(27).
SM isomère mineur : 376(0, $M^+$), 199(21), 137(36), 121(35), 105(24), 95(59), 85(100), 81(42).

d) Une solution dans le THF (120 ml) de l'acétate préparé sous c) (15 g, 0,036 mmole) a été ajoutée goutte à goutte pendant 45 min à une bouillie agitée de $LiAlH_4$ (2,08g, 0,055 mole) dans THF (140 ml) à reflux sous $N_2$. Après 10 min à reflux, le mélange refroidi a été traité successivement avec $H_2O$ (2 ml), NaOH aqueux à 20% (2 ml) et $H_2O$ (12 ml). Une filtration, suivie de concentration et distillation sous vide ont fourni deux fractions principales. La fraction 1 (5,1 g; p. éb. 60-73°C/6,7 Pa) a fourni, après chromatographie sur colonne [silica gel (160 g); toluène/acétate d'éthyle 9:1, suivi de $CH_3OH$], 0,91 g (rend. 11%) de 4-méthyl-6-(2,6,6-triméthyl-1-cyclohexén-1-yl)-2,3-hexadién-1-ol et la fraction 2 (7,4 g ; p. éb. 100-108°C/6,7 Pa) a fourni, après chromatographie sur colonne [silica gel (360 g); toluène/acétate d'éthyle 9:1], 5,5 g (rend. 65%) de ce même composé, qui se présentait sous

forme d'une huile incolore.

P. éb. (distillation au four à boules) 150-180°C (temp. du bain)/7 Pa
$R_f$ (cyclohexane/acétate d'éthyle 7:3) : 0,41
IR($CHCl_3$) : 3560, 3400 (large), 2920, 2850, 1944, 1460, 1370, 1358, 1108, 1072, 990 $cm^{-1}$
RMN($^1H$, 360MHz, $D_2O$) : 0,98(s, 6H) ; 1,41(2H) ; 1,56(2H) ; 1,58(s, 3H) ; 1,74(d, J=3Hz, 3H) ; 1,90(t large, J=7Hz, 2H) ; 1,99(2H) ; 2,08(2H) ; 4,08(d, J=5Hz, 2H) ; 5,30(m, 1H) δppm.
RMN($^{13}C$) : 200,0(s) ; 137,0(s) ; 127,4(s) ; 103,6(s) ; 91,6(d) ; 61,1(t) ; 39,9(t) ; 35,0(s) ; 34,4(t) ; 32,8(t) ; 28,6 (2q) ; 27,1(t) ; 19,8(q) ; 19,6(t) ; 19,2(q) δppm.
SM : 234(2, $M^+$), 219(5), 145(36), 133(29), 121(39), 107(46), 95(100), 81(70).

e) Une solution de 4-méthyl-6-(2,6,6-triméthyl-1-cyclohexén-1-yl)-2,3-hexadién-1-ol (obtenu selon d); 11 g, 0,044 mole) dans le $CH_2Cl_2$ (30 ml) a été ajoutée goutte à goutte pendant 1 h à une bouillie agitée mécaniquement de $H_2SO_4$ aqueux à 95% (11 g, 0,11 mole) dans $CH_2Cl_2$ (110 ml) à -40°C, sous $N_2$. Le mélange rouge foncé a encore été agité pendant 3 h à -40°C et ensuite versé lentement sur une solution froide de $NaHCO_3$ (20 g) dans $H_2O$ (250 ml) qui a été agitée pendant 30 min à température ambiante. L'émulsion blanche résultante a été partiellement concentrée (30°C/20x$10^2$ Pa) et le résidu a été extrait à l'éther. On a obtenu, après traitement, une huile orange visqueuse (11,2 g) qui a été distillée sous vide pour fournir une huile incolore (6,5 g, rend. 64%).
P. éb. 85-100°C/6,7 Pa
L'analyse chromatographique (Carbowax, toluène, $R_f$ 0,18) de ce produit à montré la présence de quatre composés dont les données analytiques étaient les suivantes:
2,3a,4,5,5aα,6,7,8,9,9a-décahydro-3aα,6,6,9aβ-tétraméthylnaphto[2,1-b] furane (5% en poids)

RMN($^1H$, 360MHz) : 0,88(s, 3H) ; 0,91(s, 3H) ; 1,13(s, 3H); 1,38(s, 3H) ; 1,00-2,20(11H) ; 4,37(dd, J=13, 3Hz, 1H) ; 4,58(d, J=13, 1,5Hz, 1H) ; 5,45(s large, 1H) δppm.
RMN($^{13}C$) : 159,4(s) ; 116,6(d) ; 86,4(s) ; 71,8(t) ; 45,3(d) ; 42,3(t) ; 40,3(t) ; 37,6(s) ; 33,5(q) ; 33,3(t) ; 26,7 (q) ; 26,0(q) ; 21,4(q) ; 19,4(t) ; 17,7(t) (1s non observé) δppm.
SM : 234(12, $M^+$), 219(100), 149(17), 123(17), 110(31), 97(69), 81(49), 69(32).

2,3a,4,5,5aα,6,7,8,9,9a-décahydro-3aα,6,6,9aα-tétraméthylnaphto[2,1-b] furane (22% en poids)

RMN($^1H$, 360MHz) : 0,93(s, 3H) ; 0,94(s, 3H) ; 1,15(s, 3H) ; 1,42(s, 3H) ; 1,00-2,20(11H) ; 4,47(dd, J=13, 3Hz, 1H) ; 4,56(d, J=13, 1,5Hz, 1H) ; 5,42(s large, 1H) δppm.
RMN($^{13}C$) : 150,7(s) ; 116,6(d) ; 86,6(s) ; 71,5(t) ; 50,5(d) ; 44,2(t) ; 39,9(t) ; 37,2(s) ; 37,0(t) ; 34,8(s) ; 33,1 (q) ; 32,1(q) ; 28,1(q) ; 26,1(q) ; 20,3(t) ; 19,0(t) δppm.
SM : 234(7, $M^+$), 219(65), 110(70), 97(100), 81(58), 69(30).

2,3a,4,5,5aβ,6,7,8,9,9a-décahydro-3aα,6,6,9aα-tétraméthylnaphto[2,1-b] furane (68% en poids)

IR($CHCl_3$) : 2950, 1460, 1380, 1140, 1100, 1060, 1020 $cm^{-1}$
RMN($^1H$, 360MHz): 0,86(s, 3H) ; 0,87(s, 3H) ; 0,94(dd, J=13, 3,5Hz, 1H) ; 1,08(s, 3H) ; 1,16(m, 1H) ; 1,38(s, 3H) ; 1,35-1,80(8H) ; 2,00(dt, J=13, 3,5Hz, 1H) ; 4,47(dd, J=13, 3,5Hz, 1H) ; 4,58(dd, J=13, 1,5Hz, 1H) ; 5,23 (s large, 1H) δppm.
RMN($^{13}C$) : 156,5(s) ; 113,1(d) ; 87,2(s) ; 72,1(t) ; 55,3(d) ; 42,2(t) ; 42,1(t) ; 38,1(t) ; 37,7(s) ; 33,6(s) ; 33,4 (q) ; 26,4(q) ; 21,5(q) ; 20,3(t) ; 19,9(q) ; 18,6(t) δppm.
SM : 234(19, $M^+$), 219(100), 191(32), 110(36), 97(66), 81(51), 69(37).

2,3a,4,5,5aβ,6,7,8,9,9a-décahydro-3aα,6,6,9aβ-tétraméthylnaphto[2,1-b] furane (3% en poids)

SM: 234(8, $M^+$), 219(40), 149(11), 110(58), 97(100), 81(32).

Les conditions de la cyclisation acide du diénol décrite ci-dessus sous e) sont des conditions standard qui, dans d'autres essais, ont été modifiées de façon connue en soi [voir, par exemple, EP-A2-403 945, inclut ici par référence], pour obtenir des mélanges plus riches en 2,3a,4,5,5aβ,6,7,8,9,9a-décahydro-3aα,6,6,9aα-tétraméthylnaphto[2,1-b] furane, voire pour obtenir ce dernier à l'état pur (pureté: 93%).

Tous ces mélanges possédaient les caractères olfactifs recherchés du composé pur, déjà décrits dans l'introduction.

Exemple 2

Préparation de 2,3a,4,5,5aβ,6,7,8,9,9a-décahydro-3aα,6 6,7α,9aα-pentaméthylnaphto[2,1-b]furane

a) Un mélange de 4-(2,5,6,6-tétraméthyl-1-cyclohexén-1-yl)-2-butanone (13 g, 0,061 mole) et d'alcool propargylique (4 g, 0,071 mole) a été ajouté goutte à goutte pendant 100 min à une bouillie de poudre de KOH (26 g, 0,46 mole; Fluka) dans le THF (100 ml), agitée mécaniquement et maintenue à une température de 20°C sous azote. Après 3 h, le mélange brun a été versé sur une solution froide de $NH_4Cl$ (28 g) dans $H_2O$ (100 ml) et les phases ont été séparées. L'extraction de la phase aqueuse avec de l'éther et chromatographie ($SiO_2$: 200 g; cyclohexane/acétate d'éthyle 7:3) a fourni le 4-méthyl-6-(2,5,6,6-tétraméthyl-1-cydohexén-1-yl)-2-hexyne-1,4-diol sous forme d'une huile visqueuse jaune pâle (12,3 g, 77%).

P. éb. (four à boules) : 180-200°C/67 Pa
$R_f$ (cyclohexane/acétate d'éthyle 7:3) : 0,14
IR($CHCl_3$): 3620, 3388 (large), 3019, 2970, 1374, 1215, 1056, 932 $cm^{-1}$
RMN($^1H$, 360MHz, $D_2O$): 0,85(s, 3H); 0,87(d, J=7Hz, 3H); 1,02(2s, 3H); 1,25-2,05(7H); 1,50(s, 3H); 1,62(s, 3H); 2,20(m, 2H); 4,30(s, 2H) δppm.
RMN($^{13}C$): 136,4(s); 127,2(s); 89,5(s); 81,7(s); 68,3(s); 50,7(t); 43,5(t); 39,4(d); 38,4(s); 31,7(t); 29,3(q); 27,3(t); 27,1(q); 23,9(t); 21,8(q); 19,9(q); 16,7(q) δppm.
SM: 264(0, $M^+$), 246(3), 231(11), 145(34), 135(82), 121(57), 107(66), 95(78), 43(100).

b) Un mélange du diol préparé sous a) (6 g, 0,022 mole), d'anhydride acétique (2,8 g, 0,027 mole), et de triéthylamine (2,8 g, 0,028 mole) a été agité à 60°C pendant 30 min sous $N_2$. Le mélange refroidi a été versé sur une solution aqueuse de HCl à 10% refroidie et extrait à l'éther. Après le traitement usuel et distillation sous vide, on a obtenu 6,8 g (rend. 98%) d'acétate de 4-hydroxy-4-méthyl-6-(2,5,6,6-tétraméthyl-1-cyclohexén-1-yl)-2-hexynyle sous forme d'une huile visqueuse jaune pâle.

P. éb. 145-148°C (temp. du bain)/7 Pa
$R_f$ (cyclohexane/acétate d'éthyle 7:3) : 0,38
IR($CHCl_3$) : 3599, 3480 (large), 3019, 2970, 1737, 1434, 1377, 1216, 1027, 965 $cm^{-1}$
RMN($^1H$, 360MHz, $D_2O$) : 0,85(s, 3H) ; 0,88(d, J=7Hz, 3H) ; 1,02(s, 3H) ; 1,25-2,30(9H) ; 1,50(s, 3H) ; 1,62(s, 3H) ; 2,10(s, 3H) ; 4,72(s, 2H) δppm.
RMN($^{13}C$) : 170,3(s) ; 136,3(s) ; 127,2(s) ; 90,6(s) ; 77,3(s) ; 68,2(s) ; 52,3(t) ; 43,5(t) ; 39,4(d) ; 38,4(s) ; 31,7(t) ; 29,3(q) ; 27,3(t) ; 27,0(q) ; 23,9(t) ; 21,8(q) ; 20,7(q) ; 19,9(q) ; 16,6(q) δppm.
SM : 306(0, $M^+$), 288(1), 231(8), 213(9), 175(20), 157(20), 145(23), 135(58), 121(52), 107(40), 95(45), 43(100).

c) Une solution d'éthyl vinyl éther (2,3 g, 0,032 mole) dans le toluène (4 ml) a été ajoutée goutte à goutte pendant 5 min à une solution agitée de l'acétate préparé sous b) (6,4 g, 0,021 mole) dans le toluène (25 ml) contenant de l'acide p-toluènesulfonique (50 mg, 2,6 mmole) à -20°C sous $N_2$. Après 1 h, cette solution a été ajoutée pendant 20 min à une bouillie agitée de $LiAlH_4$ (1,6 g, 0,042 mole) dans le THF (80 ml) à 20°C sous $N_2$. Après 30 min le mélange refroidi à été traité successivement avec $H_2O$ (1,6 ml), NaOH aqueux à 20% (1,6 ml) et $H_2O$ (8 ml). Une filtration, suivie de concentration du filtrat et chromatographie ($SiO_2$ : 150 g; toluène/acétate d'éthyle 9:1, puis acétate d'éthyle) ont fourni le 4-méthyl-6-(2,5,6,6-tétraméthyl-1-cyclohexén-1-yl)-2,3-hexadién-1-ol sous forme d'une huile visqueuse incolore (3,42 g; rend. 66%)

P. éb. 108-114°C/7 Pa
$R_f$ (toluène/acétate d'éthyle 9:1) : 0,32
IR($CHCL_3$) : 3612, 3480 (large), 3018, 2970, 1373, 1215, 1008 $cm^{-1}$
RMN($^1H$, 360MHz, $D_2O$) : 0,83(s, 3H) ; 0,88(d, J=7Hz, 3H) ; 1,00(s, 3H) ; 1,25-1,60(3H) ; 1,59(s, 3H) ; 1,75(d, J=3Hz, 3H) ; 1,65-2,20(6H) ; 4,09(d, J=7Hz, 2H) ; 5,31(m, 1H) δppm.
RMN($^{13}C$) : 200,0(s) ; 137,1(s) ; 127,1(s) ; 103,5(s) ; 91,6(d) ; 61,2(t) ; 39,4(d) ; 38,2(s) ; 34,5(t) ; 31,7(t) ; 27,3(t) ; 27,0(q) ; 21,8(q) ; 19,9(q) ; 19,2(q) ; 16,7(q) δppm.
SM : 248(0, $M^+$), 147(30), 133(39), 121(55), 109(57), 95(100), 81(48), 67(57).

d) Une solution de 4-méthyl-6-(2,5,6,6-tétraméthyl-1-cyclohexén-1-yl)-2,3-hexadién-1-ol (obtenue selon c); 1,6 g, 6,3 mmole) dans le 2-nitropropane (8 ml) a été ajoutée goutte à goutte pendant 15 min à une bouillie agitée mécaniquement de $FSO_3H$ (1 ml, 0,017 mole) dans 2-nitropropane (10 ml) à -90°C, sous $N_2$. Après 15 min le

mélange violet foncé a été laissé chauffer à -30°C et ensuite versé lentement sur une solution froide de $NaHCO_3$ (6 g) dans $H_2O$ (50 ml). Après extraction à l'éther, le traitement usuel et distillation au four à boules, on a obtenu une huile jaune pâle semi-cristalline (1,2 g). Recristallisation répétée à basse température (éther de pétrole 30/50, -70°C) a fourni le 2,3a,4,5,5aβ,6,7,8,9,9a-décahydro-3aα,6,6,7α,9aα-pentaméthylnaphto[2,1-b] furane sous forme de cristaux blancs (0,79 g; rend. 50%).

P. f. 86-88°C
$R_f$ (toluène/acétate d'éthyle 19:1) : 0,33
IR($CHCL_3$) : 2971, 1456, 1378, 1215, 1136, 1056, 1014, 855 $cm^{-1}$
RMN($^1H$, 360MHz) : 0,70(s, 3H) ; 0,85(d, J=7Hz, 3H) ; 0,89(s, 3H) ; 0,92(m, 1H); 1,06(s, 3H) ; 1,20(m, 1H) ; 1,30-1,60(5H) ; 1,39(s, 3H) ; 1,68-1,78(2H) ; 2,00(m, 1H) ; 4,47(dd, J=11,5, 3Hz, 1H) ; 4,58(d, J=11,5Hz) ; 5,23 (s large, 1H) δppm.
RMN($^{13}C$) : 156,6(s) ; 113,3(d) ; 87,0(s) ; 72,0(s) ; 56,1(d) ; 42,6(d) ; 42,0(t) ; 37,9(t) ; 36,8(s) ; 29,3(q) ; 27,4 (t) ; 26,3(q) ; 20,4(t) ; 19,8(q) ; 16,5(q) ; 16,3(q) δppm.
SM : 248(12, $M^+$), 233(58), 163(11), 149(43), 97(100).

Exemple 3

Préparation de (-)-(3aR)-1,2,3a,4,5,7,8,9,9a,9bβ-décahydro-3aα,6,6,9aα-tétraméthylnaphto[2,1-b]furane

a) Une solution de (-)-(3aS)-1,2,3a,4,5,5aβ,6,7,8,9,9a,9bβ-dodécahydro-3aα,6,6,9aα-tétraméthylnaphto[2,1-b]furan-4-one ($[\alpha]^{22}D$ = -149° ; c = 0,877, $CHCl_3$ ; 3,94 g, 15,8 mmole) dans le chloroforme (80 ml) a été traitée goutte à goutte avec une solution de brome 0,5M (35 ml, 1,1 eq.) dans le chloroforme. Le mélange a été maintenu sous agitation à température ambiante pendant la nuit jusqu'à disparition complète de la furanone de départ (chromatographie en couche mince). On a ajouté 300 ml d'éther et le tout a été lavé avec une solution aqueuse à 10% de $NaHSO_3$. Le traitement usuel a fourni 5,5 g de produit brut qui a été chauffé sous argon dans la DMF (diméthyl formamide, Fluka puriss., 95 ml), en présence de $Li_2CO_3$ (5,82 g, 78,8 mmole) et LiBr (5,48 g, 63 mmole), pendant la nuit à 100°C. Après traitement à l'éther, on a obtenu 4,05 g de produit cristallin. Ce dernier a été recristallisé deux fois dans l'hexane pour fournir 2,22 g (rend. 57%) de (-)-(3aS)-1,2,3a,4,6,7,8,9,9a,9bβ-décahydro-3aα, 6,6,9aα-tétraméthylnaphto[2,1-b] furan-4-one (pureté: 99%)

P.f. 139-140°C
$[\alpha]^{20}D$ = -166,9° (c = 0,85, $CHCl_3$)
IR($CHCl_3$) : 1700, 1600, 1240, 1160, 1120, 1060 $cm^{-1}$
UV($C_2H_5OH$) : $\lambda_{max}$ = 238 nm, ε = 12000
RMN($^1H$, 360MHz, $CDCl_3$) : 1,17(s, 3H) ; 1,23(s, 3H) ; 1,24(s, 3H) ; 1,33(s, 3H) ; 3,89(dxdxd, J=8, 8, 8Hz, 1H) ; 4,07(dxdxd, J=8, 8, 3Hz, 1H) ; 5,92(s, 1H) δppm.
SM : 248(2, $M^+$), 233(4), 164(100), 149(43), 108(21), 93(12), 43(19).

La furanone de départ peut être obtenue comme il est décrit par M. Gonzalez-Sierra et al., Heterocycles, 26, 2801 (1987).

b) Un mélange de l'énone préparée sous a) (1200 mg, 4,8 mmole), hydrate d'hydrazine (1200 mg) et éthanol anhydre (24 ml) a été porté à reflux sous argon pendant 24 h. On a concentré à pression réduite et soumis à distillation azéotropique 2 fois avec du benzène. L'hydrazone brute a été dissoute dans diéthylène glycol distillé et on a ajouté 840 mg d'hydroxyde de potassium. On a chauffé à 190°C pour distiller les volatiles. Lorsque toute l'hydrazone était disparue (30 min), le mélange de la réaction a été refroidi et extrait avec un mélange pentane/ éther 1:1. Après le traitement usuel, le résidu a été distillé par évaporation à 110-120°C/1,3 Pa pour fournir le produit désiré pur à 85% (870 mg). Une séparation chromatographique sur colonne de silica gel (Lobar B), en utilisant comme éluant un mélange hexane/éther 95:5, a fourni 650 mg (rend. 57%) de (-)-(3aR)-1,2,3a, 4,6,7,8,9,9a,9bβ-décahydro-3aα,6,6,9aα-tétraméthylnaphto[2,1-b]furane (pureté: > 98%) qui a été recristallisé dans l'hexane pour fournir un échantillon pur à plus de 99%.

P.f. 60,5-61,5°C
$[\alpha]^{20}D$ = -96,1° (c = 1,00, $CHCl_3$)
Seuil de détection : 0,17 ppb.
RMN($^1H$, 360MHz) : 1,06(s, 3H) ; 1,08(s, 3H) ; 1,12(s, 3H) ; 1,13(s, 3H) ; 2,24(d, J=4Hz, 2H) ; 3,85(dxdxd, J=8, 8, 8Hz, 1H) ; 3,98(dxdxd, J=8, 8, 4Hz, 1H) ; 5,36(t, J=4Hz, 1H) δppm.

SM : 234(4, $M^+$), 219(11), 150(58), 135(84), 84(100), 43(68).

Exemple 4

Composition parfumante

On a préparé une composition parfumante de base pour un désodorisant corporel par mélange des ingrédients suivants:

| Ingrédients | Parties en poids |
|---|---|
| Acétate de benzyle | 60 |
| Acétate de géranyle | 30 |
| Acétate de linalyle | 250 |
| Aldéhyde méthyl nonylique à 10% * | 125 |
| Glycolate d'allyle amyle | 125 |
| Astrotone ® 1) | 250 |
| Citral pur | 60 |
| Essence de citron | 370 |
| Citronellol | 60 |
| Coumarine | 370 |
| Dihydromyrcenol ® 2) | 870 |
| Estragol | 370 |
| Eugénol | 60 |
| Isobutylquinoleïne 3) à 10% * | 60 |
| Essence de lavandin | 1860 |
| Lilial ® 4) | 430 |
| Lyral ® 5) | 100 |
| Mousse cristal | 155 |
| Hédione ® 6) | 400 |
| Essence de Patchouli | 745 |
| Phénéthylol | 60 |
| Salicylate d'amyle | 430 |
| Salicylate de benzyle | 1230 |
| Sandela ® 7) | 750 |
| Essence d'Ylang | 155 |
| Vertofix coeur 8) | 370 |
| 2,4-Diméthyl-3-cyclohexène-1-carbaldéhyde à 10% * 9) | 155 |
| Total | 9900 |

* dans le dipropylène glycol

1) origine : Rhone-Poulenc.

2) 2,6-diméthyl-7-octén-2-ol; origine: International Flavors & Fragrances Inc., USA.

3) 6-(1-méthylpropyl)quinoléine; origine: International Flavors & Fragrances Inc., USA.

4) 3-(4-tert-butyl-1-phényl)-2-méthylpropanal; origine : Givaudan-Roure, Vernier, Suisse.

5) 4-(4-hydroxy-4-méthylpentyl)-3-cyclohexène-1-carboxaldéhyde ; origine: International Flavors & Fragrances Inc., USA.

6) dihydrojasmonate de méthyle; origine: Firmenich SA, Genève, Suisse.

7) 3-(isocamphyl-5)-cyclohexan-1-ol (mélange isomérique); origine: Givaudan-Roure, Vernier, Suisse.

8) origine: International Flavors & Fragrances Inc., USA.

9) origine: Firmenich SA, Genève, Suisse.

A 99 parties en poids de cette composition de base de type ambré, herbacé, floral, on a ajouté séparément 1 partie en poids d'Ambrox ® DL pour obtenir une composition A, 1 partie en poids de 2,3a,4,5,5aβ,6,7,8,9,9a-décahydro-3aα,6,6,9aα-tétraméthylnaphto[2,1-b]furane pour obtenir une nouvelle composition B et 1 partie en poids de (-)-(3aR)-1,2,3a,4,6,7,8,9,9a,9bβ-décahydro-3aα,6,6,9aα-tétraméthylnaphto[2,1-b]furane pour obtenir une nouvelle composition C.
Ces trois compositions ont ensuite été évaluées à l'aveugle par un panel de 9 experts parfumeurs.

De l'avis de ces derniers, la nouvelle composition B possédait une odeur dont la note ambrée de tête était exaltée par rapport à celle de la composition A. Elle possédait aussi moins de volume que cette dernière, mais sa note avait un caractère boisé plus marqué que celui de l'odeur de la composition A.
D'autre part, l'odeur de la composition C était moins ambrée que celle de la composition A, mais bien plus boisée et surtout beaucoup plus tenace sur la peau.

## Revendications

1. Composé de formule

(I)

possédant une double liaison dans l'une ou l'autre des positions indiquées par les lignes pointillées et dans laquelle le symbole R représente un atome d'hydrogène ou un radical méthyle.

2. 2,3a,4,5,5aβ,6,7,8,9,9a-Décahydro-3aα,6,6,9aα-tétraméthylnaphto [2,1-b]furane ou tout mélange contenant une quantité prépondérante de ce composé accompagné par des quantités mineures de l'un ou plusieurs de ses isomères.

3. (-)-(3aR)-1,2,3a,4,6,7,8,9,9a,9bβ-Décahydro-3aα,6,6,9aα-tétraméthylnaphto[2,1-b]furane ou le 2,3a, 4,5,5aβ, 6,7,8,9,9a-décahydro-3aα,6,6,7α,9aα-pentaméthylnaphto[2,1-b]furane

4. Utilisation d'un composé selon l'une des revendications 1 à 3 à titre d'ingrédient parfumant.

5. Composition parfumante ou article parfumé, comprenant un composé selon l'une des revendications 1 à 3.

6. Article parfumé selon la revendication 5, sous forme d'un parfum ou d'une eau de toilette, d'un savon, d'un shampoing, d'une crème ou d'une lotion après-shampoing, d'un gel de douche ou bain, d'un désodorisant corporel ou d'air ambiant, d'une préparation cosmétique, d'un détergent ou adoucissant textile, ou d'un produit d'entretien.

7. Procédé pour la préparation d'un composé de formule

(Id)

dans laquelle le symbole R représente un atome d'hydrogène ou un radical méthyle, caractérisé en ce qu'on cyclise à l'aide d'un agent acide, un allénol de formule

(II)

dans laquelle R a le sens indiqué ci-dessus.

**8.** Composé de formule

(II)

dans laquelle R représente un atome d'hydrogène ou un radical méthyle.

## Patentansprüche

**1.** Verbindung der Formel

(I)

welche eine Doppelbindung in der einen oder der anderen der durch punktierte Linien angegebenen Stellungen besitzt und worin das Symbol R für ein Wasserstoffatom oder einen Methylrest steht.

**2.** 2,3a,4,5,5aβ,6,7,8,9,9a-Dekahydro-3aα,6,6,9aα-tetramethylnaphtho[2,1-b]furan oder jede Mischung, welche eine überwiegende Menge dieser Verbindung enthält, begleitet von kleinen Mengen eines oder mehrerer seiner Isomeren.

**3.** (-)-(3aR)-1,2,3a,4,6,7,8,9,9a,9bβ-Dekahydro-3aα,6,6,9aα-tetramethylnaphtho[2,1-b]furan oder 2,3a,4,5,5aβ,6,7,8,9,9a-Dekahydro-3aα,6,6,7α,9aα-pentamethylnaphtho[2,1-b]furan.

**4.** Verwendung einer Verbindung gemäss einem der Patentansprüche 1 bis 3 als Riechstoff.

**5.** Riechstoffzusammensetzung oder parfümierter Artikel, enthaltend eine Verbindung gemäss einem der Patentansprüche 1 bis 3.

**6.** Parfümierter Artikel gemäss Patentanspruch 5 in Form eines Parfüms oder eines Eau de Toilette, einer Seife, eines Shampoos, einer Creme oder Lotion zur Anwendung nach der Haarwäsche, eines Dusch- oder Badegels, eines Körperdesodorans oder Raumluftverbesserers, einer kosmetischen Zubereitung, eines Detergens oder Textilweichmachers oder eines Universalhaushaltsreinigers.

**7.** Verfahren zur Herstellung einer Verbindung der Formel

(Id)

worin das Symbol R für ein Wasserstoffatom oder einen Methylrest steht, dadurch gekennzeichnet, dass man mit

Hilfe eines sauren Reagens ein Allenol der Formel

(II)

worin R die oben angegebene Bedeutung besitzt, zyklisiert.

8. Verbindung der Formel

(II)

worin R für ein Wasserstoffatom oder einen Methylrest steht.

## Claims

1. A compound of formula

(I)

having a double bond in one of the positions indicated by the dotted lines and wherein symbol R represents a hydrogen atom or a methyl radical.

2. 2,3a,4,5,5aβ,6,7,8,9,9a-Decahydro-3aα,6,6,9aα-tetramethylnaphtho[2,1-b] furan or any mixture containing a preponderant amount of the former together with minor amounts of one or several of its isomers.

3. (-)-(3aR)-1,2,3a,4,6,7,8,9,9a,9bβ-Decahydro-3aα,6,6,9aα-tetramethyl naphtho[2,1-b]furan or 2,3a,4,5,5aβ, 6,7,8,9,9a-decahydro-3aα,6,6,7α,9aα-pentamethyl naphtho[2,1-b]furan.

4. Use of a compound according to any of claims 1 to 3 as perfuming ingredient.

5. A perfuming composition or a perfumed article containing a compound according to any of claims 1 to 3.

6. A perfumed article according to claim 5, in the form of a perfume or a Cologne, a soap, a shampoo, a hair conditioner, a bath or shower gel, a body or air deodorant, a cosmetic preparation, a detergent or a fabric softener, or a household product.

7. A process for the preparation of a compound of formula

(Id)

wherein symbol R represents a hydrogen atom or a methyl radical, characterized in that an allenol of formula

(II)

where R is defined as above, is cyclized by means of an acidic agent.

**8.** A compound of formula

(II)

wherein R represents a hydrogen atom or a methyl radical.